# EUROPÄISCHE PATENTSCHRIFT

(11) **EP 3 733 617 B1**
(45) Veröffentlichungstag und Bekanntmachung des Hinweises auf die Patenterteilung: **18.05.2022**
(21) Anmeldenummer: 19172510.0
(22) Anmeldetag: 03.05.2019
(51) Int. Cl.: A61K 6/00

(54) **NIEDRIGSCHMELZENDE GLASKERAMIK**
LOW-MELTING GLASS CERAMIC
VITROCÉRAMIQUE À FAIBLE POINT DE FUSION

(43) Veröffentlichungstag der Anmeldung: 04.11.2020
(73) Patentinhaber: VITA-ZAHNFABRIK H. Rauter GmbH & Co. KG, 79713 Bad Säckingen (DE)
(72) Erfinder: Gödiker, Berit, 79713 Bad Säckingen (DE); Hackner, Michael, 79540 Lörrach (DE)
(74) Vertreter: dompatent von Kreisler Selting Werner - Partnerschaft von Patent- und Rechtsanwälten mbB

(56) Entgegenhaltungen:
- EP-A1- 3 366 260
- EP-A2- 0 468 435
- DE-A1-102010 035 545
- US-A- 6 121 175
- US-A1- 2010 035 215

## Beschreibung

Die vorliegende Erfindung betrifft eine Glaskeramik zur Verblendung einer dentalen Gerüststruktur, wobei sich die Glaskeramik durch einen hohen Gehalt an B₂O₃ auszeichnet, ein Verfahren zu deren Herstellung sowie deren Verwendung in der Herstellung dentaler Restaurationen.

Glaskeramiken sind Werkstoffe, die aus Glasschmelzen durch gesteuerte Kristallisation hergestellt werden. Dabei wird das Glas durch spezielle Temperaturbehandlungen in einen teils polykristallinen und teils glasigen, keramischen Zustand überführt, wodurch ein glasähnliches Produkt entsteht, dessen Eigenschaften sich aber von denen normaler Gläser unterscheiden.

Glaskeramiken finden in einer Vielzahl von technischen Gebieten Anwendung, wobei eine der bekanntesten als Kochfelder und Kochgeschirr im Haushaltsbereich liegt. Neben weiteren Anwendungen, beispielsweise als Hochleistungsreflektoren für digitale Projektoren, werden Glaskeramiken auch als Materialien bei der Herstellung von dentalen Restaurationen verwendet.

So beschreibt beispielsweise DE 197 50 794 Lithiumdisilikat-Glaskeramik-Produkte, die durch plastische Verformung unter Druck- und Wärmeeinwirkung oder spanende Bearbeitung zu geformten Dentalprodukten mit hoher Festigkeit verarbeitet werden können.

Außer für die Herstellung des Grundkörpers der dentalen Restauration können Glaskeramiken auch als Verblendmaterialien eingesetzt werden, mit deren Hilfe der natürliche Zahn aufbauend auf einer Gerüststruktur nachgebildet wird. Bei dieser Anwendung kommt es vor allem auf einen guten Verbund zwischen der Verblendung und der Gerüststruktur an, um eine stabile und langlebige dentale Restauration zu gewährleisten. Ein stabiler Verbund wird meist dadurch erreicht, dass die Eigenschaften des Verblendmaterials, insbesondere hinsichtlich der thermischen Ausdehnung, an die des Gerüstmaterials angepasst werden.

WO 2018/071408 beschreibt eine dentale Restauration mit einer Gerüststruktur auf Basis einer Lithiumdisilikat-Glaskeramik oder einer ZrO₂-basierten Keramik, die wenigstens eine Verblendbeschichtung enthält, wobei die Verblendbeschichtung mit der Gerüststruktur thermisch kompatibel ist.

EP 2 405 883 offenbart eine Zusammensetzung zur Verwendung für das Befestigen einer Zahnverblendung an einer Zahnträgerstruktur, wobei die Zusammensetzung 10 bis 55 Gew.-% Wasser und 40 bis 85 Gew.-% eines glaskeramischen Materials enthält, das 55 bis 75 Gew.-% Siliciumoxid und 8 bis 22 Gew.-% Aluminiumoxid umfasst. Die Verblendstruktur ist dabei in Bezug auf eine gesinterte Verblendung mit einem Vergrößerungsfaktor von 1,12 bis 1,9 proportional vergrößert und weist einen Wärmeausdehnungskoeffizienten von 8^{∗}10⁻⁶K⁻¹ bis 15,8^{∗}10⁻⁶K⁻¹ auf.

EP 1 000 588 betrifft eine keramische Dentalrestauration bestehend aus einer Basiskeramik auf Basis einer leucithaltigen Glaskeramik, die mit einer Dentalkeramik verblendet ist, wobei die Basiskeramik als Komponenten 40 bis 95 Gew.-% SiO₂ und 5 bis 25 Gew.-% Al₂O₃ enthält. Die Dentalkeramik hat einen linearen Wärmeausdehnungskoeffizienten _{α(20-500°C)} von 13,5^{∗}10⁻⁶K⁻¹ bis 17,0^{∗}10⁻⁶ K⁻¹ und die Basiskeramik einen linearen Wärmeausdehnungskoeffizienten _{α(20-500°C)} von 12,5^{∗}10⁻⁶K⁻¹ bis 15,5^{∗}10⁻⁶ K⁻¹, wobei der Wärmeausdehnungskoeffizient der Basiskeramik um 1,5^{∗}10⁻⁶K⁻¹ unter dem der Verblendkeramik liegt.

EP 0 544 145 betrifft einen dentalkeramischen Werkstoff zur Herstellung und Reparatur von metallkeramischem und vollkeramischem Zahnersatz mit einer Verarbeitungstemperatur unterhalb 700 °C und einem Wärmeausdehnungskoeffizienten α von 13-14^{∗}10⁻⁶ K⁻¹ zwischen 20 und 500 °C, gekennzeichnet durch die Zusammensetzung: 60 bis 65 Gew.-% SiO₂; 8,5 bis 11 Gew.-% Al₂O₃; 8 bis 12 Gew.-% K₂O; 10,5 bis 12 Gew.-% Na₂O; 0,7 bis 2 Gew.-% CaO; 0,6 bis 2 Gew.-% BaO; 0,5 bis 2,5 Gew.-% B₂O₃; 0,1 bis 0,6 Gew.-% Sb₂O₃; 0 bis 0,5 Gew.-% CeO₂; 1,2 bis 3,8 Gew.-% TiO₂; 0,8 bis 1,4 Gew.-% Li₂O und 1,2 bis 3,8 Gew.-% F₂.

Bei der Herstellung dentaler Restauration wird die Verblendstruktur dazu verwendet, die Gerüststruktur zu verdecken und der Restauration ein möglichst natürliches Aussehen zu verleihen, so dass sie sich unauffällig in das bestehende Zahnschema einfügt. Dazu wird die Verblendstruktur auf die Gerüststruktur aufgebracht und zusammen mit dieser einer Wärmebehandlung unterzogen, die zum einen für einen festen Verbund zwischen Gerüst- und Verblendstruktur sorgen soll und zum anderen dazu dient, die optischen Eigenschaften der dentalen Restauration einzustellen. Obwohl die im Stand der Technik beschriebenen Glaskeramiken in ihrem thermischen Verhalten an die gängigen Gerüstmaterialien angepasst sind, kommt es in der Praxis immer wieder vor, dass sich durch das unterschiedliche thermische Ausdehnungsverhalten der verwendeten Materialien Risse und Sprünge in der Verblendstruktur bilden, wodurch die dentale Restauration unbrauchbar wird.

Ein weiterer Nachteil der im Stand der Technik bekannten Glaskeramiken ist deren hoher Erweichungspunkt, der eine Verarbeitung erschwert und entsprechend hohe Temperaturen beim Herstellungsprozess erfordert, wodurch sich die Zeit bis zur Fertigstellung der dentalen Restauration erheblich verlängert. Ein weiterer Nachteil der genannten hochschmelzenden Verblendmaterialien ist außerdem, dass ihre Brenntemperatur den Erweichungspunkt der zur Ausbildung der Gerüststruktur verwendeten Glaskeramiken übersteigt, insbesondere in Fällen, in denen die Gerüststruktur aus Lithiumsilikat-Glaskeramiken besteht. Der Erweichungspunkt solcher Glaskeramiken liegt je nach Zusammensetzung in der Regel zwischen 780 und 840 °C. Eine Verblendung von Gerüsten aus dieser Materialgruppe, die mittlerweile weit verbreitet im Dentalmarkt ist, ist damit nicht möglich, da sich das Gerüst beim Aufbrennen des Verblendmaterials verformen würde. Auch bei ZrO₂-Gerüstmaterialien gibt es Probleme mit hochschmelzenden Verblendmaterialien. Der Erweichungspunkt von ZrO₂ liegt zwar weit oberhalb des Erweichungspunkts aller bekannten Verblendmaterialien, allerdings werden in der Dentalbranche häufig porös vorgesinterte ZrO₂-Gerüste, eingesetzt, die mit Färbeflüssigkeiten eingefärbt sind, um die Zahnfarbe des Patienten möglichst gut zu treffen. Wenn diese eingefärbten ZrO₂-Gerüste jedoch nach dem Dichtsintern nochmals auf Temperaturen oberhalb von 850 °C erhitzt werden, werden enthaltene färbende Komponenten teilweise oxidiert und es kann, je nach Zusammensetzung der verwendeten Färbeflüssigkeit, zu Abweichungen in der Farbtreue kommen.

Die Dokumente DE 10 2010 035545 A1 und US 6 121 175 beschreiben Glaskeramiken für dentale Anwendungen.

Es ist daher Aufgabe der vorliegenden Erfindung eine Glaskeramik für die Verblendung einer Gerüststruktur zur Verfügung zu stellen, die zum einen eine gute Verarbeitbarkeit aufweist und einen stabilen Verbund mit der Gerüststruktur ausbildet. Darüber hinaus soll sich die Glaskeramik durch gute optische Eigenschaften auszeichnen, die es ermöglichen, den natürlichen Farbverlauf eines Zahns nachzubilden.

Es wurde überraschend gefunden, dass diese Aufgabe durch eine Glaskeramik gelöst wird, die einen hohen Gehalt an B₂O₃ aufweist.

Ein erster Gegenstand der vorliegenden Erfindung ist daher eine Glaskeramik zur Verblendung einer dentalen Gerüststruktur, die SiO₂ in einer Menge von 60 bis 75 Gew.-%, vorzugsweise 65 bis 70 Gew.-%, und B₂O₃ in einer Menge von 6 bis 12 Gew.-%, vorzugsweise 7 bis 10 Gew.-%, enthält, jeweils bezogen auf das Gesamtgewicht der Glaskeramik.

Die erfindungsgemäße Glaskeramik zeichnet sich insbesondere durch einen vergleichsweise niedrigen Erweichungspunkt aus, wodurch eine materialschonende Verarbeitung möglich ist, was insbesondere im Hinblick auf die Temperatursensibilität der Materialien von Bedeutung ist, die für die Gerüststrukturen verwendet werden. Es wurde überraschend gefunden, dass dank der mit der niedrigen Erweichungstemperatur verbundenen niedrigen Verarbeitungstemperatur der Glaskeramik die sonst üblicherweise auftretende Verformung der Gerüststruktur reduziert und der Verbund zwischen Verblendkeramik und Gerüststruktur verbessert werden kann. Im Rahmen der vorliegenden Erfindung hat es sich als besonders vorteilhaft erwiesen, wenn der Erweichungspunkt der Glaskeramik nicht über 800 °C liegt. Daher weist die erfindungsgemässe Glaskeramik einen Erweichungspunkt von weniger als 790 °C auf, vorzugsweise weniger als 780 °C, besonders bevorzugt 730 bis 770 °C. Der Erweichungspunkt kann dabei mittels Erhitzungsmikroskopie bestimmt werden, wie beispielsweise in DIN 51730 dargelegt.

Die Eigenschaften der erfindungsgemäßen Glaskeramik sind insbesondere auf die Eigenschaften der zur Herstellung der Gerüststruktur verwendeten Materialien abgestimmt, um einen stabilen Verbund zwischen Gerüststruktur und Verblendkeramik zu gewährleisten. Zur Einstellung insbesondere der thermischen Eigenschaften der Glaskeramik kann diese weitere Komponenten enthalten.

In einer bevorzugten Ausführungsform weist die erfindungsgemäße Glaskeramik weiterhin K₂O in einer Menge von 6 bis 12 Gew.-%, vorzugsweise 7 bis 9 Gew.-% auf, jeweils bezogen auf das Gesamtgewicht der Glaskeramik.

In einer weiterhin bevorzugten Ausführungsform weist die Glaskeramik Al₂O₃ in einer von 3 bis 11 Gew.-%, vorzugsweise 5 bis 9 Gew.-% auf.

Ebenfalls als vorteilhaft hat sich die Anwesenheit von Na₂O erwiesen. Daher ist eine Ausführungsform bevorzugt, in der die erfindungsgemäße Glaskeramik Na₂O in einer Menge von 4 bis 11 Gew.-%, vorzugsweise 5 bis 7 Gew.-%, enthält, jeweils bezogen auf das Gesamtgewicht der Glaskeramik.

Im Gegensatz zu anderen Alkalioxiden hat sich die Anwesenheit von Li₂O in der Glaskeramik als wenig vorteilhaft erwiesen, insbesondere im Hinblick auf die Verbundhaftung mit der Gerüststruktur. Daher ist eine Ausführungsform der erfindungsgemäßen Glaskeramik bevorzugt, in der der Anteil von Li₂O in der Glaskeramik weniger als 3 Gew.-%, vorzugsweise weniger als 2 Gew.-%, besonders bevorzugt 0,1 bis 1,5 Gew.-% beträgt, jeweils bezogen auf das Gesamtgewicht der Glaskeramik.

In einer besonders bevorzugten Ausführungsform umfasst die erfindungsgemäße Glaskeramik die folgenden Komponenten:
- 60 bis 75 Gew.-%, vorzugsweise 65 bis 70 Gew.-% SiO₂;
- 6 bis 12 Gew.-%, vorzugsweise 7 bis 10 Gew.-% B₂O₃;
- 6 bis 12 Gew.-%, vorzugsweise 7 bis 9 Gew.-% K₂O;
- 3 bis 11 Gew.-%, vorzugsweise 5 bis 9 Gew.-% Al₂O₃;
- 4 bis 11 Gew.-%, vorzugsweise 5 bis 7 Gew.-% Na₂O;
- 0 bis 3 Gew.-%, vorzugsweise 0,1 bis 1,5 Gew.-% Li₂O,
wobei sich die Angaben in Gew.-% jeweils auf das Gesamtgewicht der Glaskeramik beziehen.

In einer weiterhin bevorzugten Ausführungsform weist die erfindungsgemäße Glaskeramik weniger als 0,5 Gew.-% ZnO, vorzugsweise weniger als 0,1 Gew.-% ZnO auf, jeweils bezogen auf das Gesamtgewicht der Glaskeramik.

Weiterhin bevorzugt ist eine Ausführungsform, in der die erfindungsgemäße Glaskeramik weniger als 0,5 Gew.-% ZrO₂, vorzugsweise weniger als 0,1 Gew.-% ZrO₂ aufweist, jeweils bezogen auf das Gesamtgewicht der Glaskeramik.

Dem Fachmann auf dem Gebiet der Glaskeramik ist bekannt, dass die Angabe der Komponenten der Glaskeramik in Form ihrer Oxide eine übliche Methode zur Beschreibung einer Glaskeramik ist. Nichtsdestotrotz sei an dieser Stelle klargestellt, dass die erfindungsgemäße Glaskeramik vorzugsweise aus einer Ausgangsmischung erhalten wird, die die Komponenten der Glaskeramik in Form ihrer Oxide enthält.

Die erfindungsgemäße Glaskeramik zeichnet sich dadurch aus, dass sie einen stabilen Verbund mit der Gerüststruktur ausbildet und so ein Abplatzen oder eine Beschädigung unter Belastung, beispielsweise während des Kauvorgangs, auch bei geringen Verblenddicken vermieden wird. Dieser vorteilhafte Verbund wird insbesondere dadurch erreicht, dass der Wärmeausdehnungskoeffizient WAK der Glaskeramik auf das Material der Gerüststruktur abgestimmt ist. Dabei darf der WAK der erfindungsgemäßen Glaskeramik den WAK des Gerüsts keinesfalls überschreiten. Als besonders vorteilhaft hat es sich erwiesen, wenn der WAK der erfindungsgemäßen Glaskeramik 0,1 bis 2,5^{∗}10⁻⁶K⁻¹ unterhalb des WAK der Gerüststruktur liegt. Daher ist eine Ausführungsform bevorzugt, in der die Glaskeramik einen Wärmeausdehnungskoeffizienten WAK von weniger als 9,5^{∗}10⁻⁶K⁻¹, vorzugsweise 8,3^{∗}10⁻⁶K⁻¹ bis 9,3^{∗}10⁻⁶K⁻¹ aufweist, bestimmt mittels Dilatometer.

Die erfindungsgemäße Glaskeramik zeichnet sich weiterhin durch eine hohe chemische Beständigkeit aus, was sie besonders für die Verwendung im Dentalbereich geeignet macht. Es wurde überraschend gefunden, dass die erfindungsgemäße Glaskeramik trotz des hohen Gehalts an B₂O₃ eine geringe Löslichkeit, insbesondere in sauren Milieus aufweist. Daher ist eine Ausführungsform bevorzugt, in der die erfindungsgemäße Glaskeramik eine Löslichkeit von weniger als 20 µg/cm², vorzugsweise weniger als 10 µm/cm², besonders bevorzugt 1 bis 5 µg/cm² aufweist. Die Löslichkeit kann dabei beispielsweise gemäß DIN ISO 6872 bestimmt werden.

Die Glaskeramik wird vorzugsweise in Form eines Pulvers zur Verfügung gestellt, das mit Hilfe eines flüssigen Mediums zu einer Paste verarbeitet wird, die dann auf die Gerüststruktur aufgebracht wird. Dabei hat sich überraschend gezeigt, dass die Verbundhaftung zwischen Glaskeramik und Gerüststruktur erhöht werden kann, wenn die Glaskeramik in Form eines Pulvers mit einer ausgewählten Partikelgrößenverteilung eingesetzt wird. Daher ist eine Ausführungsform bevorzugt, in der die Glaskeramik in Form eines Pulvers vorliegt, das eine Partikelgrößenverteilung D50 von 15 bis 35 µm, vorzugsweise 20 bis 25 µm aufweist, bestimmt mittels Lasergranulometer. Weiterhin bevorzugt weist die erfindungsgemäße Glaskeramik in Form eines Pulvers eine Partikelgrößenverteilung D90 von 50 bis 80 µm, vorzugsweise 60 bis 75 µm auf, bestimmt mittels Lasergranulometer. In einer bevorzugten Ausführungsform weist die erfindungsgemäße Glaskeramik in Form eines Pulvers eine Partikelgrößenverteilung D10 von 2 bis 10 µm, vorzugsweise 3 bis 5 µm auf, bestimmt mittels Lasergranulometer.

Insbesondere die Einstellung der Partikelgrößenverteilung der erfindungsgemäßen Glaskeramik hat sich als ein wichtiger Faktor bei der Ausbildung eines stabilen Verbunds zwischen der Gerüststruktur und der Glaskeramik erwiesen. Die Partikelgrößenverteilung kann beispielsweise dadurch erreicht werden, dass die Glaskeramik während ihrer Herstellung mehreren Mahl- und Schmelzvorgängen unterzogen wird. Daher ist ein weiterer Gegenstand der vorliegenden Erfindung ein Verfahren zur Herstellung der erfindungsgemäßen Glaskeramik, das die folgenden Schritte umfasst:
a) Herstellen eines Ausgangsglases durch Schmelzen der Grundkomponenten und Abschrecken der Schmelze in Wasser,
b) Aufmahlen des Glases aus Schritt a) unter Erhalt eines Pulvers;
c) Verpressen des Pulvers aus Schritt b) unter Erhalt eines Rohlings;
d) Wärmebehandeln des Rohlings unter Erhalt einer Glaskeramik; und
e) Aufmahlen des Rohlings aus Schritt e) unter Erhalt eines Pulvers.

Vorzugsweise erfolgt die Herstellung des Glases in Schritt a) des erfindungsgemäßen Verfahrens ausgehend von einer Ausgangsmischung, die die Komponenten der Glaskeramik in Form ihrer Oxide enthält.

Die Wärmebehandlung zur Ausbildung der Glaskeramik in Schritt d) des erfindungsgemäßen Verfahrens wird vorzugsweise bei einer Temperatur von 800 bis 900 °C, bevorzugt 820 bis 880 °C, besonders bevorzugt 830 bis 850 °C. durchgeführt.

Die erfindungsgemäße Glaskeramik ist insbesondere für die Herstellung von Verblendstrukturen geeignet, die auf Gerüststrukturen aufgebracht werden, um eine dentale Restauration zu erhalten. Daher ist ein weiterer Gegenstand der vorliegenden Erfindung die Verwendung der erfindungsgemäßen Glaskeramik zur Verblendung einer Gerüststruktur, vorzugsweise einer keramischen Gerüststruktur auf Basis von Lithiumdisilikat oder ZrO₂.

Um einen stabilen Verbund zwischen der Verblendstruktur und der Gerüststruktur zu erreichen, hat es sich als vorteilhaft erwiesen, wenn die Verblendstruktur und die Gerüststruktur einen ähnlichen Wärmeausdehnungskoeffizienten aufweisen. Daher ist eine Ausführungsform bevorzugt, in der die Differenz zwischen dem Wärmeausdehnungskoeffizienten der Verblendstruktur WAK_{VK} und dem Wärmeausdehnungskoeffizienten der Gerüststruktur WAK_{G} nicht mehr als 2,5^{∗}10⁻⁶K⁻¹, vorzugsweise weniger als 1,5^{∗}10⁻⁶K⁻¹, besonders bevorzugt weniger als 1,0^{∗}10⁻⁶K⁻¹, wobei die Gerüststruktur einen höheren WAK als die Verblendstruktur aufweist und der Wärmeausdehnungskoeffizient jeweils mittels Dilatometer bestimmbar ist.

Ein weiterer Gegenstand der vorliegenden Erfindung ist eine dentale Restauration umfassend eine Gerüststruktur und eine Verblendstruktur, wobei es sich bei der Verblendstruktur um eine Glaskeramik gemäß der vorliegenden Erfindung handelt. Bei der Gerüststruktur handelt es sich vorzugsweise um eine keramische Gerüststruktur auf Basis von Lithiumdisilikat oder ZrO₂. Die Gerüststruktur kann dabei so ausgebildet sein, dass sie den natürlichen Farbverlauf eines Zahns nachbildet. Auf diese Weise wird ein aufwendiges Einfärben der Verblendstruktur vermieden. Das Einfärben der Gerüststruktur kann dabei beispielsweise durch das Einbringen färbender Oxide oder mittels Färbelösungen erfolgen. Hier hat sich überraschend gezeigt, dass die optischen Eigenschaften des Gerüstes durch das Aufbrennen der Verblendstruktur nicht beeinflusst werden. Daher ist eine Ausführungsform bevorzugt, bei der die Gerüststruktur einen Farbverlauf aufweist. Alternativ ist eine Ausführungsform bevorzugt, bei der die Gerüststruktur gefärbt ist.

Es wurde überraschend gefunden, dass mit Hilfe der erfindungsgemäßen Glaskeramik ein stabiler Verbund zwischen Verblendstruktur und Gerüststruktur auch bei geringen Dicken der Verblendstruktur erreicht werden kann. Daher ist eine Ausführungsform bevorzugt, in der die Dicke der Verblendstruktur 0,2 bis 3 mm, vorzugsweise 0,5 bis 1,5 mm beträgt.

Um diese geringen Dicken zu erreichen, hat es sich als vorteilhaft erwiesen, wenn die Verblendstruktur in Form einer Paste auf die Gerüststruktur aufgebracht wird. Daher ist ein weiterer Gegenstand der vorliegenden Erfindung eine Paste umfassend ein flüssiges Medium und die erfindungsgemäße Glaskeramik. Vorzugsweise handelt es sich bei dem flüssigen Medium um Wasser, das gegebenenfalls weitere Komponenten enthalten kann.

Ein weiterer Gegenstand der vorliegenden Erfindung ist ein Verfahren zur Herstellung einer dentalen Restauration, bei dem eine Glaskeramik gemäß der vorliegenden Erfindung oder eine Paste gemäß der vorliegenden Erfindung auf eine Gerüststruktur aufgebracht wird. In einer bevorzugten Ausführungsform handelt es sich bei der Gerüststruktur um eine keramische Gerüststruktur, insbesondere auf Basis von Lithiumdisilikat oder ZrO₂.

Die vorliegende Erfindung wird anhand der folgenden Beispiele näher erläutert, wobei diese keinesfalls als Einschränkung des Erfindungsgedankens zu verstehen sind.

Beispiele:
Die erfindungsgemäße Glaskeramik wurde als Verblendmaterial auf verschiedene Gerüststrukturen aufgebracht und die Temperaturwechselbeständigkeit gemäß DIN EN ISO 9693-2:2016-07 getestet. Dazu wurden die verblendeten Gerüststrukturen abwechselnd im Ofen erwärmt und dann in Eiswasser abgeschreckt, wobei die Ofentemperatur nach jedem Abschrecken um 15 °C erhöht wurde. Die Haltezeit im Ofen betrug jeweils 30 Minuten und die Probenkörper wurden nach jedem Abschrecken auf Rissbildungen und Abplatzungen untersucht. Die Ergebnisse sind in den folgenden Tabellen zusammengefasst.

Die erfindungsgemäßen Glaskeramiken wiesen dabei einen Anteil von B₂O₃ von 8 Gew.-% auf. Als Vergleich wurden herkömmliche Glaskeramiken mit einem Anteil an B₂O₃ von 1 Gew.-% (Vgl. 1) beziehungsweise 5 Gew.-% (Vgl. 2) herangezogen.

**Tabelle 1: Temperaturwechselbeständigkeit (DIN EN ISO 9693-2:2016-07) auf Lithiumdisilikat als Gerüstmaterial**

| | erfindungsgemäße Glaskeramik | Vgl. 1 | Vgl. 2 |
|---|---|---|---|
| Sprünge bei 105 °C | keine | keine | keine |
| Sprünge bei 120 °C | keine | keine | keine |
| Sprünge bei 135 °C | keine | keine | ja |
| Sprünge bei 150 °C | keine | keine | ja |
| Sprünge bei 165 °C | keine | ja | ja |
| unbeschädigte Probenkörper | 7/7 | 5/7 | 0/7 |

**Tabelle 2: Temperaturwechselbeständigkeit ZrO₂-Gerüsten**

| | erfindungsgemäße Glaskeramik | Vgl. 1 | Vgl. 2 |
|---|---|---|---|
| Sprünge bei 105 °C | keine | keine | keine |
| Sprünge bei 120 °C | keine | keine | keine |
| Sprünge bei 135 °C | keine | ja | ja |
| Sprünge bei 150 °C | keine | keine | ja |
| Sprünge bei 165 °C | ja | ja | - |
| unbeschädigte Proben körper | 5/7 | 1/7 | 0/7 |

Weiterhin wurde die Verbundhaftung der Verblendmaterialien mit Gerüstmaterialien auf Basis von ZrO₂ mittels des Ablöse-/Rissbeginn-Prüfung (DIN EN ISO 9693-2:2016-07) bestimmt. Die Ergebnisse sind in Tabelle 3 zusammengefasst.

**Tabelle 3:**

| | Mittelwert [MPa] | Standardabweichung [MPa] |
|---|---|---|
| erfindungsgemäße Glaskeramik | 45,5 | 5,9 |
| Vgl. 1 | 32,4 | 6,3 |
| Vgl. 2 | 36,8 | 4,8 |

Wie aus der Tabelle ersichtlich, weist die erfindungsgemäße Glaskeramik eine hervorragende Verbundhaftung auf. Der Erweichungspunkt der erfindungsgemäßen Glaskeramik wurde mittels Erhitzungsmikroskopie auf 762 °C bestimmt.

## Patentansprüche

1. Glaskeramik zur Verblendung einer dentalen Gerüststruktur, **dadurch gekennzeichnet, dass** die Glaskeramik SiO₂ in einer Menge von 60 bis 75 Gew.-%, vorzugsweise 65 bis 70 Gew.-%, und B₂O₃ in einer Menge von 6 bis 12 Gew.-%, vorzugsweise 7 bis 10 Gew.-% enthält, jeweils bezogen auf das Gesamtgewicht der Glaskeramik, **dadurch gekennzeichnet, dass** die Glaskeramik einen Erweichungspunkt unterhalb von 790 °C, bestimmt gemäß Erhitzungsmikroskop, aufweist.

2. Glaskeramik gemäß Anspruch 1, **dadurch gekennzeichnet, dass** die Glaskeramik einen Erweichungspunkt unterhalb von 780 °C, besonders bevorzugt von 730 bis 770 °C aufweist, bestimmt gemäß Erhitzungsmikroskop.

3. Glaskeramik gemäß einem oder beiden der Ansprüche 1 und 2, **dadurch gekennzeichnet, dass** die Glaskeramik weiterhin K₂O in einer Menge von 7 bis 12 Gew.-%, vorzugsweise 6 bis 9 Gew.-% aufweist, bezogen auf das Gesamtgewicht der Glaskeramik.

4. Glaskeramik gemäß wenigstens einem der vorangehenden Ansprüche, **dadurch gekennzeichnet, dass** die Glaskeramik weiterhin Al₂O₃ in einer Menge von 3 bis 11 Gew.-%, vorzugsweise 5 bis 9 Gew.-% aufweist, bezogen auf das Gesamtgewicht der Glaskeramik.

5. Glaskeramik gemäß wenigstens einem der vorangehenden Ansprüche, **dadurch gekennzeichnet, dass** die Glaskeramik weiterhin Na₂O in einer Menge von 4 bis 11 Gew.-%, vorzugsweise 5 bis 7 Gew.-% aufweist, bezogen auf das Gesamtgewicht der Glaskeramik.

6. Glaskeramik gemäß wenigstens einem der vorangehenden Ansprüche, **dadurch gekennzeichnet, dass** die Glaskeramik weiterhin Li₂O in einer Menge von weniger als 3 Gew.-%, vorzugsweise weniger als 2 Gew.-%, besonders bevorzugt 0,1 bis 1,5 Gew.-% aufweist, bezogen auf das Gesamtgewicht der Glaskeramik.

7. Glaskeramik gemäß wenigstens einem der vorangehenden Ansprüche, **dadurch gekennzeichnet, dass** die Glaskeramik einen Wärmeausdehnungskoeffizienten WAK von weniger als 9,5^{∗}10⁻⁶K⁻¹, vorzugsweise 8,3 ^{∗}10⁻⁶K⁻¹ bis 9,3 ^{∗}10⁻⁶K⁻¹ aufweist, bestimmt mittels Dilatometer.

8. Glaskeramik gemäß wenigstens einem der vorangehenden Ansprüche, **dadurch gekennzeichnet, dass** die Glaskeramik eine Löslichkeit von weniger als 20 µg/cm², vorzugsweise weniger als 10 µm/cm², besonders bevorzugt 1 bis 5 µg/cm² aufweist, bestimmt gemäß DIN ISO 6872.

9. Verfahren zur Herstellung einer Glaskeramik gemäß wenigstens einem der Ansprüche 1 bis 8, **dadurch gekennzeichnet, dass** das Verfahren die folgenden Schritte umfasst:
a) Herstellen eines Ausgangsglases durch Schmelzen der Grundkomponenten und Abschrecken der Schmelze in Wasser,
b) Aufmahlen des Glases aus Schritt a) unter Erhalt eines Pulvers;
c) Verpressen des Pulvers aus Schritt b) unter Erhalt eines Rohlings;
d) Wärmebehandeln des Rohlings unter Erhalt einer Glaskeramik; und
e) Aufmahlen des Rohlings aus Schritt e) unter Erhalt eines Pulvers.

10. Verwendung einer Glaskeramik gemäß wenigstens einem der Ansprüche 1 bis 8 zur Verblendung einer dentalen Gerüststruktur, vorzugsweise einer keramischen Gerüststruktur auf Basis von Lithiumdisilikat oder ZrO₂, **dadurch gekennzeichnet, dass** die Differenz zwischen dem Wärmeausdehnungskoeffizienten der Verblendstruktur WAK_{VK} und dem Wärmeausdehnungskoeffizienten der Gerüststruktur WAK_{G} weniger als 1,5^{∗}10⁻⁶K⁻¹ beträgt, wobei der Wärmeausdehnungskoeffizient jeweils gemäß Dilatometrie bestimmbar ist

11. Verwendung gemäß Anspruch 10, **dadurch gekennzeichnet, dass** die Differenz zwischen dem Wärmeausdehnungskoeffizienten der Verblendstruktur WAK_{VK} und dem Wärmeausdehnungskoeffizienten der Gerüststruktur WAK_{G} weniger als 1^{∗}10⁻⁶K⁻¹, beträgt, wobei der Wärmeausdehnungskoeffizient jeweils gemäß Dilatometrie bestimmbar ist.

12. Dentale Restauration umfassend eine Gerüststruktur und eine Verblendstruktur, **dadurch gekennzeichnet, dass** es sich bei der Verblendstruktur um eine Glaskeramik nach wenigstens einem der Ansprüche 1 bis 8 handelt.

13. Dentale Restauration gemäß Anspruch 11, **dadurch gekennzeichnet, dass** die Dicke der Verblendstruktur 0,2 bis 3 mm, vorzugsweise 0,5 bis 1,5 mm beträgt.

14. Paste umfassend ein flüssiges Medium und ein Glaskeramikpulver nach wenigstens einem der Ansprüche 1 bis 8 in Form eines Pulvers zur Verblendung einer dentalen Gerüststruktur.

15. Verfahren zur Herstellung einer dentalen Restauration, **dadurch gekennzeichnet, dass** eine Glaskeramik nach wenigstens einem der Ansprüche 1 bis 8 oder eine Paste nach Anspruch 14 auf eine Gerüststruktur aufgebracht wird.

## Claims

1. A glass ceramic for veneering a dental framework structure, **characterized in that** said glass ceramic contains SiO₂ in an amount of from 60 to 75% by weight, preferably from 65 to 70% by weight, and B₂O₃ in an amount of from 6 to 12% by weight, preferably from 7 to 10% by weight, respectively based on the total weight of the glass ceramic, **characterized in that** said glass ceramic has a softening point of lower than 790 °C, as determined according to a heating microscope.

2. The glass ceramic according to claim 1, **characterized in that** said glass ceramic has a softening point of lower than 780 °C, more preferably from 730 to 770 °C, as determined according to a heating microscope.

3. The glass ceramic according to one or both of claims 1 and 2, **characterized in that** said glass ceramic further contains K₂O in an amount of from 7 to 12% by weight, preferably from 6 to 9% by weight, based on the total weight of the glass ceramic.

4. The glass ceramic according to at least one of the preceding claims, **characterized in that** said glass ceramic further contains Al₂O₃ in an amount of from 3 to 11% by weight, preferably from 5 to 9% by weight, based on the total weight of the glass ceramic.

5. The glass ceramic according to at least one of the preceding claims, **characterized in that** said glass ceramic further contains Na₂O in an amount of from 4 to 11% by weight, preferably from 5 to 7% by weight, based on the total weight of the glass ceramic.

6. The glass ceramic according to at least one of the preceding claims, **characterized in that** said glass ceramic further contains Li₂O in an amount of less than 3% by weight, preferably less than 2% by weight, more preferably from 0.1 to 1.5% by weight, based on the total weight of the glass ceramic.

7. The glass ceramic according to at least one of the preceding claims, **characterized in that** said glass ceramic has a coefficient of thermal expansion, CTE, of less than 9.5^{∗}10⁻⁶K⁻¹, preferably from 8.3^{∗}10⁻⁶K⁻¹ to 9.3^{∗}10⁻⁶K⁻¹, as determined by using a dilatometer.

8. The glass ceramic according to at least one of the preceding claims, **characterized in that** said glass ceramic has a solubility of less than 20 µg/cm², preferably less than 10 µg/cm², more preferably from 1 to 5 µg/cm², as determined according to DIN ISO 6872.

9. A process for preparing a glass ceramic according to at least one of claims 1 to 8, **characterized in that** said process comprises the following steps:
a) preparing a starting glass by melting the base components and quenching the melt in water;
b) milling the glass from step a) to obtain a powder;
c) pressing the powder from step b) to obtain a blank;
d) thermally treating the blank to obtain a glass ceramic; and
e) milling the blank from step d) to obtain a powder.

10. Use of a glass ceramic according to at least one of claims 1 to 8 for veneering a dental framework structure, preferably a ceramic framework structure based on lithium disilicate or ZrO₂, **characterized in that** the difference between the coefficient of thermal expansion of the veneer structure, CTE_{VS}, and the coefficient of thermal expansion of the framework structure, CTE_{FS}, is less than 1.5^{∗}10⁻⁶ K⁻¹, wherein the coefficient of thermal expansion can be respectively determined by dilatometry.

11. The use according to claim 10, **characterized in that** the difference between the coefficient of thermal expansion of the veneer structure, CTE_{VS}, and the coefficient of thermal expansion of the framework structure, CTE_{FS}, is less than 1^{∗}10⁻⁶ K⁻¹, wherein the coefficient of thermal expansion can be respectively determined by dilatometry.

12. A dental restoration including a framework structure and a veneer structure, **characterized in that** said veneer structure is a glass ceramic according to at least one of claims 1 to 8.

13. The dental restoration according to claim 11, **characterized in that** the thickness of the veneer structure is from 0.2 to 3 mm, preferably from 0.5 to 1.5 mm.

14. A paste comprising a liquid medium and a glass ceramic powder according to at least one of claims 1 to 8 in the form of a powder for veneering a dental framework structure.

15. A process for preparing a dental restoration, **characterized in that** a glass ceramic according to at least one of claims 1 to 8 or a paste according to claim 14 is applied to a framework structure.

## Revendications

1. Vitrocéramique pour mettre des facettes sur une structure d'échafaudage dentaire, **caractérisée en ce que** ladite vitrocéramique contient du SiO₂ en une teneur de 60 à 75 % en poids, de préférence de 65 à 70 % en poids, et du B₂O₃ en une teneur de 6 à 12 % en poids, de préférence de 7 à 10 % en poids, chaque fois par rapport au poids total de la vitrocéramique, **caractérisée en ce que** ladite vitrocéramique présente un point de ramollissement inférieur à 790°C, déterminé au microscope chauffant.

2. Vitrocéramique selon la revendication 1, **caractérisée en ce que** ladite vitrocéramique présente un point de ramollissement inférieur à 780°C, de manière particulièrement préférée de 730 à 770°C, déterminé au microscope chauffant.

3. Vitrocéramique selon l'une ou les deux revendications 1 et 2, **caractérisée en ce que** ladite vitrocéramique contient en outre du K₂O en une teneur de 7 à 12 % en poids, de préférence de 6 à 9 % en poids, par rapport au poids total de la vitrocéramique.

4. Vitrocéramique selon au moins une des revendications précédentes, **caractérisée en ce que** ladite vitrocéramique contient en outre du Al₂O₃ en une teneur de 3 à 11 % en poids, de préférence de 5 à 89 % en poids, par rapport au poids total de la vitrocéramique.

5. Vitrocéramique selon au moins une des revendications précédentes, **caractérisée en ce que** ladite vitrocéramique contient en outre du Na₂O en une teneur de 4 à 11 % en poids, de préférence de 5 à 7 % en poids, par rapport au poids total de la vitrocéramique.

6. Vitrocéramique selon au moins une des revendications précédentes, **caractérisée en ce que** ladite vitrocéramique contient en outre du Li₂O en une teneur inférieure à 3 % en poids, de préférence inférieure à 2 % en poids, de manière particulièrement préférée de 0,1 à 1,5 % en poids, par rapport au poids total de la vitrocéramique.

7. Vitrocéramique selon au moins une des revendications précédentes, **caractérisée en ce que** ladite vitrocéramique présente un coefficient de dilatation thermique, CDT, inférieur à 9,5^{∗}10⁻⁶K⁻¹, de préférence de 8,3 ^{∗}10⁻⁶K⁻¹ à 9,3 ^{∗}10⁻⁶K⁻¹, déterminé au dilatomètre.

8. Vitrocéramique selon au moins une des revendications précédentes, **caractérisée en ce que** ladite vitrocéramique présente une solubilité inférieure à 20 µg/cm², de préférence inférieure à 10 µm/cm², de manière particulièrement préférée de 1 à 5 µg/cm², déterminée selon DIN ISO 6872.

9. Procédé pour préparer une vitrocéramique selon au moins une des revendications 1 à 8, **caractérisé en ce que** le procédé comprend les étapes suivantes consistant à
a) préparer un verre de départ en fondant les composants de base et trempage de la masse fondue dans de l'eau,
b) moudre le verre obtenu dans l'étape a) pour obtenir une poudre,
c) presser la poudre obtenue dans l'étape b) pour obtenir un corps vert,
d) traiter le corps vert à chaud pour obtenir une vitrocéramique, et
e) moudre le corps vert obtenu dans l'étape d) pour obtenir une poudre.

10. Utilisation d'une vitrocéramique selon au moins une des revendications 1 à 8 pour mettre des facettes sur une structure d'échafaudage dentaire, de préférence sur une structure d'échafaudage céramique à base de disilicate de lithium ou ZrO₂, **caractérisée en ce que** la différence entre le coefficient de dilatation thermique de la structure de facette, CDT_{VK}, et le coefficient de dilatation thermique de la structure d'échafaudage, CDT_{G}, est moins de 1,5^{∗}10⁻⁶K⁻¹, le coefficient de dilatation thermique chaque fois pouvant être déterminé par dilatométrie.

11. Utilisation selon la revendication 10, **caractérisée en ce que** la différence entre le coefficient de dilatation thermique de la structure de facette, CDT_{VK}, et le coefficient de dilatation thermique de la structure d'échafaudage, CDT_{G}, est moins de 1^{∗}10-6K-1, le coefficient de dilatation thermique chaque fois pouvant être déterminé par dilatométrie.

12. Restauration dentaire comprenant une structure d'échafaudage et une structure de facette, **caractérisée en ce que** ladite structure de facette est une vitrocéramique selon au moins une des revendications 1 à 8.

13. Restauration dentaire selon la revendication 11, **caractérisée en ce que** l'épaisseur de la structure de facette est de 0,2 à 3 mm, de préférence de 0,5 à 1,5 mm.

14. Pâte comprenant un milieu liquide et une poudre de vitrocéramique selon au moins une des revendications 1 à 8 sous la forme d'une poudre pour mettre des facettes sur une structure d'échafaudage dentaire.

15. Procédé pour préparer une restauration dentaire, **caractérisé en ce qu'**une vitrocéramique selon au moins une des revendications 1 à 8 ou une pâte selon la revendication 14 est appliquée à une structure d'échafaudage.
